# EUROPEAN PATENT APPLICATION

(11) **EP 1 245 241 A1**
(43) Date of publication of application: **02.10.2002**
(21) Application number: 01870072.4
(22) Date of filing: 30.03.2001
(51) Int. Cl.: A61L 24/04

(54) **Polymerized hydrogel adhesives comprising low amounts of residual monomers**

(71) Applicant: The Procter & Gamble Company, Cincinnati, Ohio 45202 (US)
(72) Inventor: Merrigan, Steve Ray, 65125 Pescara (IT); Goldman, Stephen Allen, 65013 Città Sant'Angelo (Pescara) (IT); Struillou, Arnaud Pierre, 13330 Pelissanne (FR)
(74) Representative: Canonici, Jean-Jacques

(57) **Abstract**

The present invention relates to polymerized hydrogel adhesives, in particular those wherein the hydrogel is formed from monomers which include acrylamido-2-methane propanesulfonic acid or salts thereof (AMPS monomers) and those which contain glycerol as a humectant. In such adhesives, the levels of unpolymerized residual monomers, such as unpolymerized AMPS, acrylonitrile, acrylamide and t-butyl acrylamide; and the levels of other impurities such as acrolein, are kept very low.

The present invention also relates to a process for making hydogel adhesives with very low levels of such unwanted materials. Such a process involves preparation of reaction mixtures under selected pH and/or temperature conditions and polymerization of AMPS monomers using selected conditions of pH and UV curing.

## Description

### Field of the Invention

The present invention relates to polymerized hydrogel adhesives which are capable of attaching to mammalian skin and can be used in various personal care products, such as waste-management articles, absorbent articles, and a variety of functional articles to be worn by a human. The hydrogels herein are characterized by very low amounts of residual monomer.

### Background of the Invention

While hydrogel body adhesives for use in consumer products such as absorbent articles and waste-management articles have previously been described in, respectively, EP 1 025 823 and EP 1 025 866, the disclosure of hydrogel adhesive has mainly occurred in the context of small volume medical applications, such as skin electrodes, transdermal drug delivery and wound healing. In EP 1 025 823 and EP 1 025 866, certain hydrogel requirements for consumer products produced on a large scale, such as absorbent and human waste-management products, are disclosed, including the need for secure attachment, painless removal and stability of adhesion in presence of excess moisture.

In addition to delivering the above-mentioned benefits, it is particularly important, especially for large-scale production of consumer products, that the hydrogel adhesives used must provide a very good safety profile.

Use of acrylamido-2-methane propanesulfonic acid or its salts (both acid and salts referred to herein as acrylamido-2-methane propanesulfonic acid or AMPS) as one of the monomers which can be polymerized to form the hydrogel is well known and are disclosed in, e.g., the documents referred to above. For hydrogel compositions containing polymerized AMPS, it has been found that not only the level of unreacted AMPS, but also the level of impurities such as acrylonitrile, acrylamide, and t-butyl acrylamide, present as monomers in the AMPS starting material, must be controlled. This is so that the level of acrylonitrile, acrylamide, and t-butyl acrylamide are kept within specifically defined target levels in the eventually resulting hydrogel composition. It has also been found that when glycerol is present as a humectant in polymerized hydrogel adhesives made by UV curing, the level of acrolein must also be controlled in the finished composition, and be kept under well defined target levels.

The present invention also provides process steps which, when used in the making of the hydrogel compositions herein, allow the above-identified residual unwanted monomers to be kept to very low levels in the final resulting hydrogel adhesive.

### Summary of the Invention

In one embodiment, the present invention relates to polymerized hydrogel adhesives which comprise 10-90 wt% water and 10-60 wt% of a cross-linked hydrophilic polymer. The hydrophilic polymer is made by polymerizing monomers which comprise acrylamido-2-methane propanesulfonic acid and/or its salts (AMPS). The resulting adhesive contains less than 100 ppb acrylonitrile and less than 100 nanograms/gram (ppb) acrylamide, preferably less than 50 ppb, more preferably less than 25 ppb and most preferably less than 10 ppb of acrylonitrile and acrylamide. The adhesive also contains at least 1% by weight of AMPS, preferably at least 10% by weight AMPS in polymerized form. Addditionally, it is preferred that the polymerized hydrogel adhesive contain less than 200 micrograms/gram (ppm), more preferably less than 100 ppm, and even more preferably less than 50 ppm of residual AMPS monomers. Additionally, it is preferred that when the polymerized hydrogel adhesive contains both AMPS and one or more co-monomers in polymerized form, that the residual level of all monomers is less than 400 ppm, preferably less than 200 ppm, even more preferably less than 100 ppm, and most preferably less than 50 ppm. Additionally it is preferred that the polymerized hydrogel adhesive contain less than 100 ppb of t-butyl acrylamide, preferably less than 50 ppb, more preferably less than 25 ppb, and even more preferably less than 10 ppb of this material.

In another embodiment, the present invention relates to polymerized hydrogel adhesives wherein the polymerization is carried out at least partly by UV photo-initiation and wherein such adhesives contain 5-80 wt% of glycerol as a humectant, preferably 10-80 wt%, most preferably 30-80 wt%, and wherein the level of acrolein is below 300 ppb, preferably below 200 ppb, most preferably below 100 ppb.

In still another embodiment of the present invention, the present invention relates to a process for making hydrogel adhesives comprising AMPS in salt form as a monomer and using a defined neutralization method to produce an aqueous reaction mixture. In such a neutralization procedure, the pH of the eaction mixture is maintained below 9, preferably at a pH below 7 and preferably the reaction mixture is maintained at a temperature below 40 °C, more preferably below 25 °C. Preferably the polymerization of said aqueous reaction mixture is conducted at a pH of 3.5 to 7, more preferably at a pH of 4 to 6.5, most preferably at a pH 4.5 to 6. The polymerization is conducted using UV curing at selected UV irradiation conditions, and with a selected photoinitiator.

### Detailed Description

The present invention relates to hydrogel adhesives which are capable of attaching to mammalian skin and are made by cross-linking and polymerization of at least one monomer to form polymer, in presence of water.

The cross-linking between polymer chains creates a 3-dimensional matrix for the polymer, also referred to as gel form or hydrogel. Physical cross linking refers to polymers having cross links which are not chemical covalent bonds but are of a physical nature such that for example there are areas in the 3 dimensional matrix having high crystallinity or areas having a high glass transition temperature or areas having hydrophobic interactions. Chemical cross linking refers to polymers which are linked by chemical bonds. The polymer can be chemically cross linked by radiation techniques such as UV-, E beam- , gamma or micro-wave radiation or by co-polymerizing the monomers with a di/poly-functional crosslinker via the use e.g., of UV, thermal and/or redox polymerization initiators. The polymer can also be ionically crosslinked.

The strong-acid monomer 2-acrylamido-2-methylpropanesulfonic acid and its salts is particularly preferred as a monomer or co-monomer for use in forming the polymer component of the polymerized adhesive hydrogel. This monomer is available e.g., from Lubrizol in the acid form or in the neutralized form (see below) as the sodium salt in concentrated aqueous solution (e.g., 50 wt% and 58 wt%). Other suitable monomers and co-monomers can be acidic, neutral, basic, or zwitterionic. Suitable strong-acid monomers include those selected from the the group of olefinically unsaturated aliphatic or aromatic sulfonic acids such as 3-sulfopropyl (meth)acrylate, 2-sulfoethyl (meth)acrylate, vinylsulfonic acid, styrene sulfonic acid, allyl sulfonic acid, vinyl toluene sulfonic acid, methacrylic sulfonic acid and the like. Particularly preferred strong-acid monomers are 2-acrylamido-2-methylpropanesulfonic acid, 3-sulfopropyl (meth)acrylate, 2-sulfoethyl (meth)acrylate. Suitable wak-acid monomers include those selected from the group of olefinically unsaturated carboxylic acids and carboxylic acid anhydrides such as acrylic acid, methacyclic acid, maleic acid, itaconic acid, crotonic acid, ethacrylic acid, citroconic acid, fumaric acid, β-sterylacrylic acid and the like. Particularly preferred weak-acid monomers are acrylic acid and methacrylic acid.

Suitable polyfunctional monomer crosslinkers include polyethyleneoxide di(meth)acrylates with varying PEG molecular weights, IRR280 (a PEG diacrylate available from UCB Chemical), trimethylolpropane ethyoxylate tri(meth)acrylate with varying ethyleneoxide molecular weights, IRR210 (an alkoxylated triacrylate; available from UCB Chemicals), trimethyolpropane tri(meth)acrylate, divnylbenzene, pentaerythritol triacrylate, pentaeythritol triallyl ether, triallylamine, N,N-methylene-bis-acrylamideand others polyfunctional monomer crosslinkers known to the art. Preferred monomer crosslinkers include the polyfunctional diacrylates and triacrylates.

Chemical crosslinking can also be effected after polymerization by use of polyfunctional reagents capable of reacting with polymer functional groups such as ethyleneglycol diglycidyl ether, polyols such as glycerol, and other polyfunctional reagents known to the art.

Crosslinking can also be effected all or in part by ionic crosslinking wherein groups of opposite charge interact via ionic interactions. Suitable ionic crosslinking agents include those known to the art including polyvalent cations such as Al⁺³ and Ca⁺², di/poly-amines, di/poly-quaternary ammonium compounds, including polymeric polyamines and polyquaternary ammonium compounds known to the art.

In a first embodiment of the invention the adhesive compositions herein use acrylamido-2-methane propanesulfonic acid or its salts (AMPS) as one of the monomers. The salts of AMPS preferably comprise a monovalent couterion, with the Na⁺ counterion being particularly preferred. It has been recognized that commercial acid AMPS and NaAMPS (typically provided as concentrated aqueous solutions) monomer feedstock contains ppm concentrations of acrylamide, acrylonitrile, and t-butyl acrylamide. Upon polymerization, the concentrations of these impurities need to be reduced as much as possible for use in products in contact with skin the levels of acrylonitrite, acrylamide, and t-butyl acrylamide in the adhesive composition should be kept at levels of below 100 ppb, preferably below 50 ppb, more preferably below 25 ppb, most preferably below 10 ppb, for a hydrogel adhesive composition containing at least 1% polymerized AMPS, preferably at least 10 wt% AMPS. Methods suitable for measuring the residual levels of AMPS and selected other monomers in the polymerized adhesive hydrogel are given in the test methods. Section. Also included in the test method section are methods suitable for measuring the residual levels of the monomer impurities, acrylamide, acrylonitrile, and t-butyl acrylamide,

It has been found that, when using commercially available 50% NaAMPS feedstocks, the concentrations of one or more of residual acrylamide, acrylonitrile, and t-butyl acrylamide in the final hydrogel typically remains too high.

To reduce the concentration of residual acrylamide and acrylonitrile, it has now been discovered that another procedure is required to prepare the concentrated (e.g., 50 wt% or 58 wt%) NaAMPS feedstock solution used to prepare monomer solutions for polymerization. Conventionally, concentrated NaAMPS feedstock is prepared by a process wherein the acid AMPS is added to a solution of concentrated base (e.g., NaOH) whereby all the acid AMPS added is neutralized by the excess base into NaAMPS and the NaAMPS formed by this neutralization is exposed for significant time periods during and/or after the neutralization process to a pH that is above 10, often well above 10 as a result of the presence of unreacted base.

It has now been found that when the NaAMPS feedstock is made by the improved process in which the base is added to a solution of AMPS in acid form (e.g., aqueous solution), with the pH being consistently kept below 9, preferably below 8, most preferably below 7 during and after the neutralization, the level of acrytonitrile and acrylamide residual monomers in the polymerized hydrogel adhesive is considerably reduced.

Without being bound by theory, it is believed that concentrated NaAMPS feedstock prepared by the conventional process described above, wherein the acid AMPS is added to a concentrated solution of base, contains conjugate addition products of NaAMPS, acrylonitrile, acrylamide, and t-butyl acrylamide (e.g., such as the hydrates shown hereinafter).

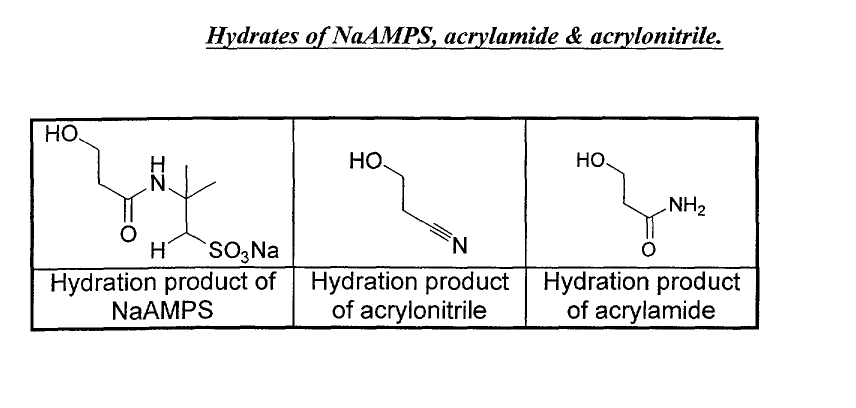

This conjugate addition reaction is believed to be facilitated at high pH, where the concentration of hydroxide ion is high, (i.e., when the concentration of base is in excess of the concentration of acid, which occurs in a neutralization procedure where the acid is added to the base). Such an addition product is no longer an olefinically unsaturated monomer and is not polymerized under the polymerization conditions used to form polymer from the AMPS monomer. It is believed that all of these addition products are capable of reverting back to the monomer to some degree, after polymerization, in the polymerized hydrogel adhesive. This results in concentrations of these monomers in the polymerized hydrogel that are greater than would be found if these conjugate addition products of these monomers were not formed during the neutralization process.

It is preferred that during the aforementioned neutralization of acid AMPS that the temperature of the AMPS solution be maintained below 40 °C, preferably below 25 °C. Without being bound by theory, it is believed that the lower temperature minimizes the premature polymerization of AMPS at low pH.

It is also preferred that, prior to neutralization, the concentrations of the monomer impurities in the acid AMPS feedstock (e.g., acrylonitrile, acrylamide, and t-butyl acrylamide) be reduced to as low as possible prior to neutralization and polymerzation. This can be accomplished by improved synthesis and/or purification methods. For example, the acid AMPS can be recrystallized to reduce the levels of acrylonitrile, acrylamide, and t-butyl acrylamide to lower initial levels. This will generally lower the level of these impurities in the polymerized adhesive hydrogel. Recrystallization can be effected one or more times from a suitable solvent (e.g., methanol) using procedures well known in the art.

In another embodiment of the present invention, is provided a hydrogel adhesive made by photoinitiation polymerization. Photoinitiation will usually be applied by subjecting the pre-gel reaction mixture of monomer(s) containing an appropriate photoinitiation agent to UV light after it has been spread or coated as a layer on siliconised release paper or other solid or porous substrate. The incident UV intensity, typically at a wavelength in the range from about 240 to about 400 nm overlaps to at least some degree with the UV absorption band of the photoinitiator and is of sufficient intensity and exposure duration (e.g., 10-3000 mW/cm²) to complete the polymerization in a reasonable time. To facilitate the process, it is often preferable to expose the reaction mixture to several UV irradiation sources, in sequence. The processing will generally be carried out in a controlled manner involving a precise predetermined sequence of mixing and thermal treatment or history.

Such free-radical photoinitiation agents or photoinitiators are well known in the art and can be present in quantities up to 5 % by weight, preferably from 0.02 % to 2 %, more preferably from 0.02 % to 0.4 %. Such photoinitiators include type α-hydroxy-ketones and benzilidimethyl-ketals . Suitable photoinitators include dimethoxybenzylphenone (available under the trade name of Irgacure 651 from Ciba Specialty Chemicals). 2-hydroxy-2-methyl-propiophenone (available under the trade name of Daracur 1173 from Ciba Specialty Chemicals), I-hydroxycyclohexylphenylketone (available under the trade name Irgacure 184 from Ciba Speciality Chemicals), diethoxyacetophenone, and 4-(2-hydroxyethoxy)phenyl-(2-hydroxy-2-methylpropyl) ketone (available under the trade name of Irgacure 2959 from Ciba Specialty Chemicals). Daracure 1173, Irgacure 2959 and Irgacure 184 are preferred photoinitiators. Irgacure 2959 and Irgacure 184 are particularly preferred. Combinations of photoinitiators can also be used. In addition, polymerization can be carried out by using thermal initiator(s) and/or redox initiator(s) well known to the art or one or more of these initiators in combination with the aforemetioned photoinitiators Suitable thermal initiators include potassium persulfate and VA044 (available from Wako). Suitable redox initiators include the combination of hydrogen peroxide and ascorbic acid and sodium persulfate and ascorbic acid.

The hydrogel compositions described herein can comprise a humectant, preferably glycerol, at levels of from 5-80% of the hydrogel. Alternate humectants well known in the art can also instead of or in combination with glycerol.

Other common additives known in the art such as polymerization inhibitors, chain transfer agents, salts, surfactants, soluble or dispersible polymers, buffers, preservatives, antioxidants, pigments, mineral fillers, and the like and mixtures thereof may also be comprised within the adhesive composition in quantities up to about 10 % by weight each respectively.

It has been discovered that during the photopolymerization process, glycerol can produce acrolein as a decomposition product, and thus it is critical that the levels of acrolein in the adhesive compositions be kept at levels below 300 ppb, preferably below 200 ppb, more preferably below 100 ppb, most preferably below 40 ppb. A method suitable for measuring the level of acrolein in a polymerized adhesive hydrogel is described in the Test Methods section.

Without being bound by theory, it is believed that acrolein can be formed by acid-catalyzed or base-catalyzed reactions of glycerol with free radicals generated during photopolymerization, wherein the concentration of free radicals are especially high. It is believed that by controlling the pH within the limits described hereinafter, the amount of acrolein generated during photopolymerization as a result of these acid or base catalyzed reactions can be diminished.

It has been found that by controlling the pH of the monomer pre-mix solution in the range of 3.5 to 7, preferably 4-6.5, more preferably 4.5-6; that the level of acrolein formed during the polymerization reaction is reduced. This is especially important to control the level of acrolein in the finished hydrogel.

Furthermore, it has been found that the wavelength of the UV-radiation should be carefully controlled during the photopolymerization reaction, to obtain optimum results on reduction of acrolein as well as acrylamide. It is preferable to minimize the relative percentage of UV irradiation reaching the monomer solution and hydrogel with wavelengths below 280 nm, preferably below 300 nm, more preferably below 320 nm, most preferably below 335 nm. This can be achieved by the use of a UV light source that has inherently low output in these wavelength ranges or by interposing one or more high-pass UV-filters between the UV light source and the monomer solution and hydrogel.

Examples of high-pass UV filters that can be used for this purpose include the Borofloat UV Filters (e.g., T320) available from Bedamfpurgs-technik. Other examples include the high-pass UV filters made by Schott GlassWerks (e.g., WG-280, WG-295, WG-305, WG-320, and WG-335). It is preferred that the integrated UV intensity in units of W/cm² in the aforementioned wavelength regions by reduced to less than 10%, preferably less than 7%, more preferably less than 4%, most preferably less than 1% of the integrated UV intensity in the entire UV region (i.e., 200-400 nm)

Without being bound by theory, it is believed that the AMPS monomer is capable of absorbing UV at the aforementioned low wavelengths and, as a result of being raised to an excited energy level, can decompose to generate acrylamide (e.g., by a Norrish Type II rearrangement).

While not being bound by theory, it is believed that by reducing the UV intensity in the low-wavelength region comprising the principle UV absorption band of the AMPS, the direct absorption of UV by the AMPS is reduced which greatly minimizes the formation of acrylamide via the photodecomposition reaction described above. This is done, while still providing UV photons in the wavelength range above about about 320 nm. UV in this wavelength range is still capable of initiating photopolymerization using the aforementioned photoinitiators, thus allowing the photo-polymerization to take place effectively even with all the radiation below 280 nm, 300 nm, 320 nm, or even 335 nm cut-off.

Without being bound by theory, it is also believed that reducing the UV irradiation in the aforementioned wavelength ranges also reduces the formation of acrolein via photodecomposition or free-radical reactions involving glycerol.

Without being bound by theory, it is believed that a second mechanism for the photodecomposition of AMPS to form acrylamide involves energy transfer from the excited triplet state of the photoinitiator -to the resulting in the photodecomposition as described above- This mechanism can occur even if the AMPS is prevented from directly absorbing UV irradiation by e.g., the use of a UV filter.

Without being bound by theory, It is believed that by using a photoinitiator that has a triplet state energy more different from the triplet energy state of AMPS, the amount of effective energy transfer from excited state photoinitiator to AMPS can be decreased. By decreasing this energy transfer, it is believed that AMPS decomposition to acrylamide is also decreased. The photoinitiators Irgacure 2959 and Irgacure 184 have been found to be particularly effective in reducing the amount of acrylamide generated, while still being effective at producing a polymerized hydrogel adhesive with low levels of residual monomers. Irgacure 2859 is particularly preferred.

### Test Methods

### 1. pH of Monomer Solutions

The pH of a monomer solution can be measured using methods well known to the art. For example, an Ionlabph/ion level 2P meter can be used equipped with a SenTix 41 electrode ( available from Wissenschaftlich Technische Werkstaetten)

### 2. Residual NaAMPS in Polymerized Hydrogels

Sample Preparation: Add 50 ml of 0.9% w/v saline solution to 0.500 g of hydrogel and put the mixture in a thermostatic bath for a minimum of 12 hours at approximately 40°C. Collect an aliquot of the supernatant through a 0.45 µm hydrophilic filter into a syringe and then transfer into a HPLC autosampler vial.

Analysis: HPLC/DAD - 20µl of the hydrogel filtrate (as above) is injected directly into the HPLC, for example a Waters Millennium 2020 C/S equipped with a Waters 600 solvent delivery module, Waters 717+ auto injector, Waters 996 photo diode array detector and a Hypersil SAS C1 250 X 4.6mm 5µm column set. The mobile phase comprises 90% of eluent A (H₂O:Acetonitrile 90:10 v/v + PIC A low UV) and 10% of eluent B (H₂O:Acetonitrile 30:70 v/v + PIC A low UV). PIC A is a Waters ion pair reagent containing buffered tetra butyl ammonium ion pair solution. The flow rate is 1 ml/min. For detection a photo diode array channel 200nm (bandwidth 1.2nm) is used, the UV Spectra across 190-300nm can be applied for peak purity assessment. The level of analyte is quantified using standard procedures well known to the art and reported as micrograms analyte per gram of hydrogel (ppm).

### 3. Residual N,N-Dimethylacrylamide in Polymerized Hydrogels

Sample Preparation: Add 50 ml of 0.9% w/v saline solution to 0.500 g of hydrogel in a capped glass container. The resulting mixture is placed in a thermostatic bath for a minimum of 12 hours at approximately 40°C. The supernatant is separated from the gel by filtration through filter paper, for example Schleicher and Schuell 597, and collected. To the filtrate, 20 ml of CH₂Cl₂ and 200µl of internal standard solution (0.0001g/l tetradecane) are added and the resulting mixture is put in an ultrasonic bath for 15 minutes. After sonication, the CH₂Cl₂ solution is separated from the aqueous layer, dried with for example Na₂SO₄ and transferred to an autosampler vial.

Analysis: 2µl of this solution is injected into a GC for example a Trace 2000 series under the following conditions:

| | |
|---|---|
| Initial Temp | 80°C |
| Initial time | 2min |
| Rate 1 | 20°C/min |
| Final temp 1 | 150°C |
| Final time 1 | 2min |
| Rate 2 | 30°C |
| Final temp 2 | 230°C |
| Final temp 2 | 10min |
| Injection mode | splitless |
| Injection temp | 250°C |
| FID Detection temp | 280°C |
| Carrier gas | He |
| Column head-pressure | 75kPa |

The level of analyte is quantified using standard procedures well known to the art and reported as micrograms analyte per gram of hydrogel (ppm).

### 4. Residual Acrylonitrile and Acrolein in Polymerized Hydrogels

Sample Preparation: Add 50 ml of 0.9% w/v saline solution to 0.500 g of hydrogel in a capped glass container. The resulting mixture is placed in a thermostatic bath for a minimum of 12 hours at approximately 40°C. The liquid is separated from the gel and collected. The headspace of this solution is analyzed as described below.

Analysis: Follow procedure outlined in U.S. EPA method 8240. The level of analyte is quantified using standard procedures well known to the art and reported as nanograms analyte per gram of hydrogel (ppb). **5. Residual Acrylamide in Polymerized Hydrogel**

Sample Preparation: Add 50 ml of 0.9% w/v saline solution to 0.500 g of hydrogel in a capped glass container, the resulting mixture is placed in a thermostatic bath for a minimum of 12 hours at approximately 40°C. The supernatant is separated from the gel and collected. The supernatant is analyzed as outlined below.

Analysis: Follow procedure outlined in U.S. EPA method 8032A. Detection is via MS in negative Cl mode with isobutane as the reactant gas.

The level of analyte is quantified using standard procedures well known to the art and reported as nanograms analyte per gram of hydrogel (ppb).

### 6. Residual Acrylic Acid in Polymerized Hydrogels

Sample Preparation: Add 50 ml of 0.9% w/v saline solution to 0.500 g of hydrogel in a capped glass container. The resulting mixture is placed in a thermostatic bath for a minimum of 12 hours at approximately 40°C. Collect the supernatant through a 0.45 µm hydrophilic filter into a syringe and then store in an HPLC autosampler vial. The filtrate is analyzed as described below.

Analysis: Follow procedure outlined in EDANA method 410.1 The level of analyte is quantified using standard procedures well known to the art and reported as micrograms analyte per gram of hydrogel (ppm).

### 7. Residual tert-Butyl Acrylamide in Polymerized Hydrogel

Sample Preparation: Add 50 ml of 0.9% w/v saline solution to 0.500 g of hydrogel in a capped glass container, the resulting mixture is placed in a thermostatic bath for a minimum of 12 hours at approximately 40°C. The supernatant is separated from the gel by filtration through filter paper, for example Schleicher and Schuell 597, and collected. To the filtrate, 20 ml of ethyl acetate and 200µl of internal standard solution (0.0001g/l 3-nitrophenol) are added and the resulting mixture is put in an ultrasonic bath for 15 minutes. After sonication the ethyl acetate solution is separated from the aqueous layer, dried with for example Na₂SO₄ and transferred to an autosampler vial.

Analysis: 2µl of this solution is injected into a GC, for example a Trace 2000 series, under the following conditions:

| | |
|---|---|
| Initial Temp | 80°C |
| Initial time | 2min |
| Rate 1 | 20°C/min |
| Final temp 1 | 150°C |
| Final time 1 | 2min |
| Rate 2 | 30°C |
| Final temp 2 | 230°C |
| Final temp 2 | 10min |
| Injection mode | splitless |
| Injection temp | 250°C |
| NPD Detection temp | 280°C |
| Carrier gas | He |
| Column head-pressure | 75kPa |

The level of analyte is quantified using standard procedures well known to the art and reported as nanograms analyte per gram of hydrogel (ppb).

### EXAMPLES

### Example 1: Preparation of 50 wt% aqueous solution of NaAMPS

Approximately 14 parts of acid AMPS (2-acrylamido-2-methyl-1-propanesulphonic acid; recrystallized one time from methanol) is added to a solution containing approximately 0.02 parts MEHQ inhibitor (4-methoxyphenol, available from Aldrich), approximately 0.44 parts potassium phosphate buffer (Aldrich), and approximately 37.35 parts distilled water and allowed to dissolve. Recrystallization reduces the levels of acrylamide, a acrylonitrile, and t-butylacrylamide to approximately 20 ppm, 5 ppm, and 50 ppm, respectively. The reaction mixture is cooled with an ice-cold water bath to maintain the temperature of the reaction mixture below approximately 25°C as approximately 5 parts of approximately 50 wt% NaOH (Aldrich) is added dropwise. This quantity of NaOH is slightly less than one equivalent relative to the level of acid AMPS. After the addition of the NaOH is complete another aliquot of approximately 14 parts Acid AMPS is dissolved in the reaction mixture before adding dropwise another approximately 5 parts of the 50 wt% NaOH. After the second addition of 50 wt% NaOH is completed another aliquot of approximately 14 parts Acid AMPS is dissolved in the reaction mixture before adding dropwise another 5 parts of the 50 wt% NaOH. A final addition of approximately 3.2 parts of acid AMPS is dissolved in the reaction mixture followed by the final dropwise addition of approximately 2.45 parts of the 50 wt% NaOH. The final pH of the mixture is adjusted to approximately pH=5 with dropwise addition of a small quantity of NaOH. This yields an approximately 50 wt% aqueous NaAMPS solution.

### Example 2: Preparation of 58 wt% aqueous solution of NaAMPS:

Approximately 17 parts of Acid AMPS (2-acrylamido-2-methyl-1-propanesulphonic acid, recrystallized one time from methanol) is added to a solution containing approximately 0.02 parts MEHQ inhibitor (4-methoxyphenol, available from Aldrich), approximately 0.51 parts potassium phosphate buffer (Aldrich), and approximately 27.32 parts distilled water and allowed to dissolve. The reaction mixture is cooled with an ice-cold water bath to maintain the temperature of the reaction mixture below approximately 25°C as approximately 6 parts of approximately 50 wt% NaOH (Aldrich) is added dropwise. The level of NaOH added is slightly less than one equivalent relative to the level of acid AMPS. After the addition of the NaOH is completed, another aliquot of approximately 17 parts Acid AMPS is dissolved in the reaction mixture before adding dropwise another approximately 6 parts of the 50 wt% NaOH. After the second addition of 50 wt% NaOH is completed another aliquot of approximately 17 parts Acid AMPS is dissolved in the reaction mixture before adding dropwise another approximately 6 parts of 50 wt% NaOH. A final addition of approximately 1.43 parts of acid AMPS is dissolved in the reaction mixture followed by the final dropwise addition of approximately 2.24 parts of 50 wt% NaOH. The final pH of the mixture is adjusted to approximately pH=5 with dropwise addition of a small quantity of NaOH. This yields an approximately 58 wt% aqueous NaAMPS solution.

### Example 3: Preparation of Polymerized Adhesive Hydrogel

A 50 wt% aqueous solution of NaAMPS is prepared by addition of NaOH to acid AMPS (recrystallized one time from methanol) using a procedure analogous to that described in Example 1. To approximately 68.4 parts of this solution, approximately 31.5 parts of glycerol is added and the resulting mixture is stirred for approximately 15 min. To 100 parts of this solution is added approximately 0.048 parts of the photoinitiator Daracur 1173 (Aldrich) and approximately 0.11 parts of cross-linker IRR 210 (a polyoxyethylene triacrylate cross-linker from UCB). The resultant mixture is dispersed and/or dissolved with stirring.

The monomer solution is spread at a basis weight of approximately 1.0 kilograms per square meter onto a thin, porous non-woven substrate. The solution is polymerized via UV irradiation curing with an IST 200 ozone-free arc lamp (Spectrum Type: CKII-OF). A high-pass UV filter with a frequency cut-off of approximately 320 nM (UV Filter Borofloat T320 from Bedampfungs-Technik) is positioned between the lamp and the sample to filter out low-frequency UV irradiation. The solution-coated substrate is irradiated while passing underneath the lamp on a variable-speed belt positioned approximately 13 cm underneath the lamp. The speed of the belt is set at approximately 7 meter/min. The peak output power of the lamp is measured using an UMD-1 power meter (Eta Plus Electronic) and the output intensity of the lamp is adjusted so that the incident peak UV power on the sample is approximately 600 milliwatt/cm² (measured with the UV filter). Twelve consecutive passes of the sample underneath the lamp is used to polymerize the monomer solution and convert it into a soft adhesive hydrogel with low levels of residual NaAMPS, acrylonitrile, acrylamide, and acrolein (see Table).

### Example 4: Preparation of Polymerized Adhesive Hydrogel

To a solution of approximately 22.4 parts of the approximately 58 wt% NaAMPS solution of Example 2 and approximately 13.2 parts of distilled water, approximately 19.2 parts of acrylic acid is added. To this solution approximately 6.4 parts of 50 wt% NaOH (Aldrich) is added dropwise with constant stirring, while maintaining the temperature to less than approximately 25°C with an ice bath. The NaOH that is added is sufficient to convert approximately 30 mole% of the acrylic acid to sodium acrylate. Approximately 38.9 parts of glycerol (Agar) is added and the resulting mixture is stirred for 15 min. The solution is covered to shield it from light.

To one aliquot of this solution (4-1), approximately 0.13 parts of the polyfunctional cross-linker IRR210 and 0.30 parts of Daracur 1173 is added to approximately 100 parts of the monomer solution and dispersed and/or dissolved with stirring for approximately 15 minutes. To a second aliquot of this solution (4-2), approximately 0.13 parts of the polyfunctional cross-linker IRR210 and 0.30 parts of Irgacure 2959 is added to approximately 100 parts of the monomer solution and dispersed and/or dissolved with stirring for approximately 15 minutes.

The monomer solutions are spread at a basis weight of approximately 1.0 kilograms per square meter onto siliconized release paper (for example, CO.GE.SIL."Silfraft - 70gr" (Palazzo)), that has been surface treated by wiping with a very-thin layer of Pluronic 6400 surfactant (BASF) to facilitate spreading of the solution. For handling purposes, the release paper is pre-positioned inside a 8.5 cm diameter polystyrene Petri dish.

The monomer solutions are polymerized via UV irradiation curing as described in Example 3 The peak output power of the lamp is measured using an UMD-1 power meter (Eta Plus Electronic) and the output intensity of the lamp is adjusted so that the incident peak UV power on the sample is approximately 900 milliwatt/cm² (measured with the UV filter). Twelve consecutive passes of the sample underneath the lamp is used to polymerize the monomer solutions and convert them into soft adhesive hydrogels. It is evident that the use of of Irgacure 2959 significantly reduces the level of residual acrylamide in the polymerized hydrogel, without increasing significantly the residual levels of the starting monomers (see Table)

### Example 5: Preparation of Polymerized Adhesive Hydrogel

To a solution of approximately 22.4 parts of the approximately 58 wt% NaAMPS solution of Example 2 and approximately 13.2 parts of distilled water, approximately 19.2 parts of acrylic acid is added. To this solution approximately 6.4 parts of 50 wt% NaOH (Aldrich) is added dropwise with constant stirring, while maintaining the temperature to less than approximately 25°C with an ice bath. The NaOH that is added is sufficient to convert approximately 30 mole% of the acrylic acid to sodium acrylate. Approximately 38.9 parts of glycerol (Agar) is added and the resulting mixture is stirred for 15 min. The solution is covered to shield it from light.

Prior to polymerization, a mixture of approximately 0.13 parts of the polyfunctional cross-linker IRR210 and 0.23 parts of Daracur 1173 is added to approximately 100 parts of the monomer solution and dispersed and/or dissolved with stirring for approximately 15 minutes. The monomer solution is spread at a basis weight of approximately 1.0 kilograms per square meter onto siliconized release paper as described in Example 4.

The monomer solution is polymerized as described in Example 4 The peak output power of the lamp is measured using an UMD-1 power meter (Eta Plus Electronic) and the output intensity of the lamp is adjusted so that the incident peak UV power on the sample is approximately 900 milliwatt/cm² (measured without the UV filter). Nine consecutive passes of the sample underneath the lamp is used to polymerize the monomer solution and convert it into a soft adhesive hydrogel with low levels of residual NaAMPS, acrylic acid, acrylonitrile, acrylamide, and acrolein. (see Table)

### Example 6: Preparation of Polymerized Adhesive Hydrogel

A 58 wt% aqueous solution was prepared according to Example 2, but using non-recrystallized acid AMPS (Aldrich) and adjusting the final pH to 4.0. The non-recrystallized acid AMPS has levels of acrylamide, acrylonitrile, and t-butylacrylamide of approximately 400 ppm, 50 ppm, and 500 ppm, respectively. Approximately 49.1 parts of this NaAMPS solution and approximately 15.7 parts of N,N-dimethylacrylamide (NN-DMA; Aldrich) are combined with stirring. To this solution is added approximately 35 parts of glycerol (Agar). The resultant solution is stirred for approximately 10 minutes and covered to shield it from light. Prior to polymerization, approximately 0.11 parts of Daracur 1173 and approximately 0.06 parts of IRR-210 are added to approximately 100 parts of the monomer solution and dispersed and/or dissolved with stirring for approximately 15 minutes.

The monomer solution is spread at a basis weight of approximately 1.0 kilograms per square meter onto siliconized release paper as described in Example 4. The monomer solution is polymerized via UV irradiation curing as described in Example 4, except that a Schott 335 nm high-pass UV filter is used and the incident peak UV power on the sample is approximately 1800 milliwatt/cm² (measured without the UV filter). Twelve consecutive passes of the sample underneath the lamp is used to polymerize the monomer solution and convert it into a soft adhesive hydrogel with low levels of residual NaAMPS, N,N-dimethylacrylamide, acrylonitrile, acrylamide, and acrolein (see Table)

### Example 7: Preparation of Polymerized Adhesive Hydrogel

A 50 wt% aqueous solution of NaAMPS is prepared by addition of NaOH to acid AMPS (unrecrystallized; Aldrich) using a procedure analogous to that described in Example 1,. To approximately 68.4 parts of this solution, approximately 31.46 parts of glycerol is added and the resulting mixture is stirred for approximately 15 min. One aliquot of this solution is adjusted with NaOH to pH=5.25 (7-1). A second aliquot is adjusted with NaOH to pH=7 (7-2). To 100 parts of each solution is added approximately 0.04 parts of the photoinitiator Daracur 1173 and approximately 0.11 parts of cross-linker IRR 210. The resultant mixture is dispersed and/or dissolved with stirring.

The monomer solutions are spread at a basis weight of approximately 1.0 kilograms per square meter onto siliconized release paper as described in Example 4. The solution is polymerized via UV irradiation curing as described in Example 4, except a high-pass UV filter with a frequency cut-off of approximately 335 nm (Schott filter) is positioned between the lamp and the sample to filter out low-frequency UV irradiation. The peak output power of the lamp is measured using an UMD-1 power meter (Eta Plus Electronic) and the output intensity of the lamp is adjusted so that the incident peak UV power on the sample is approximately 1800 milliwatt/cm² (measured without the UV filter). Twelve consecutive passes of the sample underneath the lamp is used to polymerize the monomer solutions and convert them into soft adhesive hydrogels. It is evident that the use of pH=5.25 significantly reduces the level of acrolein in the polymerized hydrogel, without increasing significantly the residual levels of the starting monomers or the residual levels of monomer impurities (see Table)

| **Residual Levels of Monomers and Impurities in Polymerized Hydrogels** | | | | | | |
|---|---|---|---|---|---|---|
| Hydrogel Example # | NaAMPS *(ppm) | Acrylic Acid *(ppm) | NN-DMA* (ppm) | Acrylamide * (ppb) | Acrylonitrile* (ppb) | Acrolein* (ppb) |
| 3 | <100 | - | - | <45 | <50 | <100 |
| 4-1 | <100 | <10 | - | 500 | <50 | 1.5 |
| 4-2 | <100 | 25 | - | <45 | <50 | 1.5 |
| 5 | 100 | 60 | - | <45 | <50 | 190 |
| 6 | <100 | - | 7.5 | <45 | <50 | <100 |
| 7-1 | <100 | - | - | <45 | <50 | 100 |
| 7-2 | <100 | - | - | <45 | <50 | 570 |

| | | | | | | |
|---|---|---|---|---|---|---|
| * Using the methods described in the test methods section, the detection limits for NaAMPS, Acrylic Acid, NN-DMA, acrylonitrile, and acrolein are 100 ppm, 10 ppm, 100 ppb, 45 ppb, 50 ppb, and 100 ppb, respectively. When the level of analyte measured is less than the detection limits, the value is reported as being less than the detection limit. | | | | | | |

## Claims

1. A hydrogel adhesive comprising 10-90 wt% water and 10-60 wt% of cross-linked hydrophilic polymer made from monomers comprising acrylamido-2-methanepropanesulfonic acid and salts thereof, **characterized in that** said adhesive contains at least 1 wt% polymerized acrylamido-2-methanepropanesulfonic acid or salts thereof, and further contains less than 100 ppb acrylonitrile, and less than 100 ppb acrylamide.

2. A hydrogel adhesive according to Claim 1 comprising less than 50 ppb, preferably less than 25 ppb, and more preferably less than 10 ppb acrylonitrile, and also comprises less than 50 ppb, preferably less than 25 ppb, and more preferably less than 10 ppb acrylamide.

3. A hydrogel adhesive according to Claims 1 or Claim 2 which comprises at least 10 wt% of polymerized acrylamido-2-methanepropanesulfonic acid or salt thereof.

4. A hydrogel adhesive according to any of Claims 1 to 3 which contains less than 200 ppm, preferably less than 100 ppm, more preferably less than 50 ppm of residual acrylamido-2-methanepropanesulfonic acid or salt monomer and less than 100 ppb of t-butyl acrylamide.

5. A co-polymerized hydrogel adhesive according to any of Claims 1 to 4 which comprises acrylamido-2-methanepropanesulfonic acid or salt therof co-polymerized with one or more additional co-monomers, said co-polymerized hydrogel adhesive containing less than 400 ppm, preferably less than 200 ppm, more preferably less than 100 ppm, and even more preferably less than 50 ppm of residual monomers.

6. A co-polymerized hydrogel adhesive according to Claim 5 wherein said additional co-monomer is acrylic acid or salt thereof.

7. A hydrogel adhesive according to any of Claims 1-6 wherein the polymerization of monomers and/or co-monomers is carried out at least partly by UV photoinitiation.

8. A hydrogel adhesive according to Claim 7 which comprises 5-80 wt% of glycerol as a humectant, and a photoinitiator, wherein said hydrogel adhesive contains less than 300 ppb of acrolein.

9. A hydrogel adhesive according to Claim 8 which comprises 10-80 wt% glycerol, preferably 30-80 wt% glycerol and wherein the level of acrolein is below 200 ppb, preferably below 100 ppb, even more preferably below 40 ppb.

10. A hydrogel adhesive comprising 10-85 wt% water, 10-60 wt% of crosslinked hydrophilic polymer, and 5-80 wt% of glycerol, wherein the hydrophilic polymer has been formed by polymerization carried out at least partly by UV photoinitiation, said hydrogel adhesive containing less than 300 ppb acrolein and less than 400 ppm of residual monomers.

11. A hydrogel adhesive according to Claim 10 comprising 10-80 wt%, preferably 30-80 wt%, of glycerol and containing less than 200 ppb, preferably less than 100 ppb, most preferably less than 40 ppb acrolein and less than 200 ppm, preferably less than 100 ppm, most preferably less than 50 ppm of residual monomers.

12. A process for making a hydrogel adhesive comprising the steps of:
- providing a first aqueous solution of acrylamido-2-methaneproponesulfonic acid ;
- providing a second aqueous solution of base, the quantity of said base being chosen to neutralize said first aqueous solution ;
- making an aqueous reaction mixture by mixing said first aqueous solution and said second aqueous solution, said mixing being carried out by adding said second aqueous solution to first second aqueous solution, the pH of said aqueous reaction mixture being kept below 9 during the making of said aqueous reaction mixture; and
- polymerizing said aqueous reaction mixture.

13. A process according to Claim 12 wherein the pH of the aqueous reaction mixture is kept below 7.

14. A process according to Claim 12 or Claim 13 wherein the temperature of said aqueous reaction mixture is kept below 40 °C, and preferably below 25 °C.

15. A process according to any of Claims 12 to 14 wherein the polymerization of said aqueous reaction mixture is conducted at a pH 3.5 to 7, preferably 4 to 6.5, more preferably 4.5-6.

16. A process according to any of Claims 12 - 15 wherein the polymerization is conducted by UV curing, and the integrated UV intensity at wavelengths less than 280 nm, preferably less than 300 nm, more preferably less than 320 nm, most preferably less than 335 nm is less than 10%, preferably less than 7%, even more preferably less than 4%, most preferably less than 1% of the total integrated UV intensity with wavelengths less than 400 nm.

17. A process according to Claim 16 which utilizes a photoinitiator selected from Danacur 1173, Irgacure 2959, Irgacure 500, and Irgacure 184 to initiate polymerization.

18. A process according to the Claim 17 wherein the photoinitiator is Irgacure 2959.
